**Europäisches Patentamt**

**European Patent Office**

**Office européen des brevets**

(19)

(11) Publication number : **0 456 317 B1**

(12)

# EUROPEAN PATENT SPECIFICATION

(45) Date of publication of patent specification :
25.08.93 Bulletin 93/34

(51) Int. Cl.[5] : **B01J 31/24**, **C08G 67/02**

(21) Application number : **91201062.6**

(22) Date of filing : **03.05.91**

(54) Catalyst compositions and polymerization process, hexakisphosphines and hexahalo compounds.

(30) Priority : **10.05.90 NL 9001114**

(43) Date of publication of application :
**13.11.91 Bulletin 91/46**

(45) Publication of the grant of the patent :
**25.08.93 Bulletin 93/34**

(84) Designated Contracting States :
**BE CH DE ES FR GB IT LI NL**

(56) References cited :
**EP-A- 0 380 162**
**US-A- 4 183 825**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Keijsper, Johannes Jacobus**
**Badhuisweg 3**
**NL-1031 CM Amsterdam (NL)**
Inventor : **van Leeuwen, Petrus Wilhelmus Nicolaas Maria**
**Badhuisweg 3**
**NL-1031 CM Amsterdam (NL)**
Inventor : **van der Made, Alexander Willem**
**Badhuisweg 3**
**NL-1031 CM Amsterdam (NL)**

Jouve, 18, rue Saint-Denis, 75001 PARIS

## Description

The invention relates to novel catalyst compositions which are suitable for use in the preparation of polymers of carbon monoxide with one or more olefinically unsaturated compounds.

It is known that linear polymers of carbon monoxide with one or more olefinically unsaturated compounds, in which polymers the units originating from carbon monoxide on the one hand and the units originating from the olefinically unsaturated compounds on the other hand occur in a substantially alternating arrangement, can be prepared by contacting the monomers at elevated temperature and pressure with a solution of a catalyst composition in a diluent in which the polymers are insoluble or virtually insoluble, which catalyst composition contains a Group VIII metal and a bis- or tetrakisphosphine of the general formula $(R^1R^2P)_2R^3$ or $(R^1R^2P)_4R^4$, in which $R^1$ and $R^2$ represent identical or different optionally polar-substituted hydrocarbon groups, in which $R^3$ is a divalent organic linking group which connects the two phosphorus atoms with each other and has a structure such that there are at least two carbon atoms between the two phosphorus atoms and in which $R^4$ is a quadrivalent organic linking group which connects the four phosphorus atoms with each other and has a structure such that there are at least two carbon atoms between each two phosphorus atoms.

During the polymerization the polymers are obtained in the form of a suspension in the diluent. Hitherto, the preparation of the polymers has mainly been done batchwise. Batchwise preparation of the polymers takes place by introducing the catalyst composition into a reactor containing diluent and monomers and which is at the desired temperature and pressure. As polymerization proceeds, the pressure drops, the concentration of the polymers in the diluent increases and the viscosity of the suspension rises. As a rule, polymerization is continued until the viscosity of the suspension has reached such a high value that continuation of the process would create difficulties, inter alia in connection with heat removal. If desired, the batchwise polymer preparation can be carried out keeping not only the temperature constant but also the pressure constant by adding monomers to the reactor during the polymerization. The polymerization is, as a rule, terminated by cooling the reaction mixture to room temperature and releasing the pressure. The polymer suspension is then drawn out of the reactor and the reactor is washed with diluent.

A problem with the above-described polymer preparation is reactor fouling. During the polymerization, some of the formed polymers deposit on reactor components below the surface of the liquid, such as the reactor wall, the baffles, the stirrer shaft, the stirrer blades, the cooling and heating coils and the dip tubes. These polymer deposits remain in the reactor after the polymer suspension has left the reactor and cannot be removed by washing the reactor with diluent. In some cases the reactor fouling can be very serious and can in extreme cases amount to about 50%, which means that only about 50% of the prepared polymer leaves the reactor in the form of a suspension, while about 50% remains behind as a deposit on the reactor components. This phenomenon can form a serious obstacle to the application of the polymerization on a commercial scale.

During an investigation by the Applicant into this subject, it was recently found that reactor fouling can be combated by, before contacting the monomers with the catalyst solution, suspending a solid material in the diluent in a quantity given by the formula a $\geqq$ 100xbxc, where a is the number of grams of the solid material per litre diluent, b is the number of metres of the average particle size of the solid material and c is the number of kg/m$^3$ of the bulk density of the solid material.

In the course of continued research by the Applicant into this subject, another measure has now been found by which reactor fouling can be effectively combated, which measure can be applied alone or in combination with the above-described measure relating to the suspension of a solid material in the diluent. The measure now found is in essence that in the catalyst compositions containing a bisphosphine of the general formula $(R^1R^2P)_2R^3$ or a tetrakisphosphine of the general formula $(R^1R^2P)_4R^4$ the bisphosphine or tetrakisphosphine is replaced by a hexakisphosphine of the general formula $(R^1R^2P)_6R^5$ in which $R^1$ and $R^2$ have the meanings indicated above and $R^5$ is a hexavalent organic linking group which links the six phosphorus atoms with each other and has a structure such that there are at least two carbon atoms between each two phosphorus atoms.

Catalyst compositions which contain a Group VIII metal and a hexakisphosphine of the general formula $(R^1R^2P)_6R^5$ are novel.

The present patent application therefore relates to novel catalyst compositions which contain a Group VIII metal and a hexakisphosphine of the general formula $(R^1R^2P)_6R^5$ in which $R^1$ and $R^2$ represent identical or different optionally polar-substituted hydrocarbyl groups and in which $R^5$ is a hexavalent organic linking group which connects the six phosphorus atoms with each other and has a structure such that there are at least two carbon atoms between each two phosphorus atoms.

The patent application further relates to the use of these catalyst compositions in the preparation of polymers of carbon monoxide with one or more olefinically unsaturated compounds.

A number of the hexakisphosphines of the general formula $(R^1R^2P)_6R^5$ which are eligible for use in the catalyst compositions of the invention are, moreover, novel compounds. The present patent application also relates to these novel compounds and to their preparation. The hexahalides obtained as byproducts in the preparation of the novel hexakisphosphines are also novel compounds. The patent application finally relates to these novel hexahalides and their preparation.

Group VIII metals are understood in this patent application to be the noble metals ruthenium, rhodium, palladium, osmium, iridium and platinum, as well as the iron group metals iron, cobalt and nickel. In the catalyst compositions of the invention, the Group VIII metal is preferably selected from palladium, nickel and cobalt. Palladium is particularly preferred as Group VIII metal. The Group VIII metal is preferably incorporated in the catalyst compositions in the form of a salt of a carboxylic acid, in particular in the form of an acetate. In addition to a Group VIII metal and a hexakisphosphine, the catalyst compositions of the invention also preferably contain an anion of an acid with a pKa of less than 4 and in particular an anion of an acid with a pKa of less than 2. Examples of acids with a pKa of less than 2 are mineral acids such as sulphuric acid and perchloric acid, sulphonic acids such as methanesulphonic acid, trifluoromethanesulphonic acid and para-toluenesulphonic acid and halogen carboxylic acids such as trichloroacetic acid, difluoroacetic acid and trifluoroacetic acid. A sulphonic acid such as para-toluenesulphonic acid and a halogen carboxylic acid such as trifluoroacetic acid are preferred. The anion can be incorporated in the catalyst either in the form of a compound from which the desired anion splits off or in the form of a mixture of compounds from which the desired anion is formed by interaction. As a rule, the anion is incorporated in the catalyst compositions in the form of an acid. If desired, the anion can also be incorporated in the catalyst compositions in the form of a main group metal salt or a non-noble transition metal salt of the corresponding acid. If an anion of a carboxylic acid is chosen, its incorporation in the catalyst compositions can be in the form of the acid or in the form of a derivative thereof, such as an alkyl or aryl ester, an amide, an imide, an anhydride, an ortho-ester, a lactone, a lactam or an alkylidene dicarboxylate. The anion is preferably present in the catalyst compositions in a quantity of 1-100 and in particular 2-50 mol per g.atom of Group VIII metal. Besides its application as a separate component, the anion of an acid with a pKa of less than 4 can also be present in the catalyst because, for example, palladium trifluoroacetate or palladium para-tosylate was used as Group VIII metal compound.

Besides a Group VIII metal, a hexakisphosphine and, optionally, an anion of an acid with a pKa of less than 4, the catalyst compositions of the invention may also contain an organic oxidizing agent. Examples of suitable organic oxidizing agents are 1,2- and 1,4-quinones, aliphatic nitrites such as butyl nitrite and aromatic nitro compounds such as nitrobenzene and 2,4-dinitrotoluene. 1,4-benzoquinone and 1,4-naphthoquinone are preferred. The quantity of organic oxidizing agent used preferably amounts to 5-5,000 and in particular 10-1,000 mol per g.atom Group VIII metal.

In the catalyst compositions of the invention, the hexakisphosphine is preferably present in a quantity of 0.1-1.0 and in particular 0.2-0.5 mol per g.atom Group VIII metal. In the hexakisphosphines the groups $R^1$ and $R^2$ are preferably identical aryl groups which contain at least one alkoxy substituent at the ortho position relative to the phosphorus atom to which the aryl group is linked. An alkoxy-substituted aryl group of this kind will be referred to further in this patent application as an $R^6$ group. Examples of $R^6$ groups are the 2-methoxyphenyl group, the 2,4-dimethoxyphenyl group, the 2,6-dimethoxyphenyl group and the 2,4,6-trimethoxyphenyl group. Hexakisphosphines are preferred in which the $R^6$ group is a 2-methoxyphenyl group. Hexakisphosphines are also preferred in which the $R^5$ linking group consists of a benzene ring having as substituents on the 1-, 3- and 5-positions a (-$CH_2$)$_2$CH-O- $\overset{*}{C}$ $H_2$- group (2-(methyleneoxy)trimethylene) which is linked to the benzene ring via the (methyleneoxy) carbon atom labelled with * and which benzene ring optionally contains methyl groups as substituents at the 2-, 4- and 6-positions.

Hexakisphosphines of the general formula $[(R^6)_2P]_6R^5$ in which the linking group $R^5$ has the meaning indicated above are novel. The present patent application also relates to these novel compounds and to the preparation thereof. The preparation of these compounds can take place by the reaction of an alkali metal diaryl phosphide of the general formula $MP(R^6)_2$ in which M represents an alkali metal such as sodium, with a hexahalogen compound consisting of a benzene ring containing as substituent at the 1-, 3- and 5-positions a $(CH_2X)_2CH$-O-$CH_2$- group in which X represents a halogen such as chlorine or bromine and which benzene ring can, if desired, contain methyl groups as substituents at the 2-, 4- and 6-positions. The alkali metal diaryl phosphides required for the synthesis of the novel hexakisphosphines can be obtained by the reaction in liquid ammonia of an alkali metal M with a trisphosphine of the general formula $(R^6)_3P$. The preparation of the halogen compounds required for the synthesis of the novel hexakisphosphines can take place starting from mesitylene by converting this compound by halogenation, for example with N-bromosuccinimide, into 1,3,5-tris(halomethyl)benzene and then reacting this compound with an epihalohydrin such as epibromohydrin. The 1,3,5-tris[(1,3-dihalo-2-propoxy)methyl]benzenes obtained are novel. The patent application also relates to these novel compounds and to their preparation. The halogen compounds needed for the preparation of the novel hexakisphosphines can also be prepared by halomethylation of mesitylene with, for example, paraformaldehyde and hydrobromic acid, followed by reaction of the thus obtained 1,3,5-tris(halomethyl)-2,4,6-trimethylbenzene with an epihalohydrin such as epibromohydrin. The 1,3,5-tris[(1,3-dihalo-2-propoxy)methyl]-2,4,6-trimethylbenzenes obtained are novel. The present patent application also relates to these novel compounds and to their preparation.

As novel compounds according to the invention, the following compounds can be mentioned:

a) 1,3,5-tris[(1,3-dibromo-2-propoxy)methyl]benzene that was prepared by the reaction of 1,3,5-tris(bromomethyl)benzene with epibromohydrin,

b) 1,3,5-tris[(1,3-dibromo-2-propoxy)methyl]-2,4,6-trimethylbenzene that was prepared by the reaction of 1,3,5-tris(bromomethyl)-2,4,6-trimethylbenzene with epibromohydrin,

c) 1,3,5-tris[(1,3-bis(bis(2-methoxyphenyl)phosphino)-2-propoxy)methyl]benzene that was prepared by reacting sodiumdi(2-methoxyphenyl)phosphide, obtained by the reaction in liquid ammonia of sodium with tris(2-methoxyphenyl)phosphine, with the novel hexahalo compound mentioned under a), and

d) 1,3,5-tris[(1,3-bis(bis(2-methoxyphenyl)phosphino)-2-propoxy)methyl]-2,4,6-trimethylbenzene that was prepared by reacting sodiumdi(2-methoxyphenyl)phosphide, obtained as mentioned under c), with the novel hexahalo compound mentioned under b).

The polymerization using the catalyst compositions of the invention is carried out by contacting the monomers at elevated temperature and pressure with a solution of the catalyst composition in a diluent in which the polymers are insoluble or almost insoluble. As diluent, lower alcohols such as methanol are very suitable. As olefinically unsaturated compounds that, using the catalyst compositions of the invention, can be polymerized with carbon monoxide, compounds consisting exclusively of carbon and hydrogen as well as compounds which, in addition to carbon and hydrogen, contain one or more hetero-atoms are eligible. The catalyst compositions according to the invention are preferably applied for the preparation of polymers of carbon monoxide with one or more olefinically unsaturated hydrocarbons. Examples of suitable hydrocarbon monomers are ethene, propene, butene-1, hexene-1, octene-1, styrene, cyclopentene, norbornene and dicyclopentadiene. The catalyst compositions of the invention are in particular very suitable for application in the preparation of copolymers of carbon monoxide with ethene and in the preparation of terpolymers of carbon monoxide with ethene and with an $\alpha$-olefin, in particular propene.

The quantity of catalyst composition used in the preparation of the polymers may vary within wide limits. Per mol of olefinically unsaturated compound to be polymerized a quantity of catalyst composition is used which preferably contains $10^{-7}$ to $10^{-3}$ and in particular $10^{-6}$ to $10^{-4}$ g.atom Group VIII metal.

The preparation of the polymers is preferably carried out at a temperature of 25 to 150 °C and a pressure of 2 to 150 bar and in particular at a temperature of 30 to 130 °C and a pressure of 5 to 100 bar. The molar ratio of the olefinically unsaturated compounds relative to carbon monoxide in the mixture to be polymerized is preferably 10:1 to 1:10 and in particular 5:1 to 1:5.

The invention will now be illustrated with reference to the following Examples.

Example 1

1,3,5-tris(bromomethyl)benzene was prepared as follows. A mixture of 0.5 mol mesitylene, 1.6 mol N-bromosuccinimide, 50 mg dibenzoyl peroxide and 1.3 litre tetrachloromethane was boiled under reflux while being stirred and irradiated with a 375 Watt photographic lamp. After cooling and filtration, the reaction mixture was washed successively with an aqueous sodium bicarbonate solution and twice with water, dried above magnesium sulphate and concentrated to 400 ml. Petroleum ether with a boiling range between 60 and 85 °C was then added and the mixture was stored at 0 °C. The crystals that were formed were filtered off and purified by repeated crystallization from a mixture of trichloromethane and petroleum ether. Yield of 1,3,5-tris(bromomethyl)benzene: 40 g (23%).

Example 2

1,3,5-tris(bromomethyl)-2,4,6-trimethylbenzene was prepared as follows. To a mixture of 12.0 g (0.10 mol) mesitylene, 10.0 g (0.33 mol) paraformaldehyde and 50 ml 99.7% glacial acetic acid was rapidly added 70 ml of a 31% solution of hydrobromic acid in acetic acid. The mixture was heated in a period of 1 hour to 90 °C and maintained at this temperature for 8 hours. After cooling the mixture, the solid material was filtered off and washed with pentane. Yield of 1,3,5-tris(bromomethyl)-2,4,6-trimethylbenzene was 36.3 g (91%). By using the mother liquor in a subsequent preparation, the yield could be enhanced to almost 100%.

Example 3

1,3,5-tris[(1,3-dibromo-2-propoxy)methyl]benzene was prepared as follows. A mixture of 24.6 g (0.18 mol) epibromohydrin, 20.22 g (0.0567 mol) of the 1,3,5-tris(bromomethyl)benzene prepared according to Example 1 and 20 mg mercury dichloride was heated for 9 hours at 160 °C. After adding 3.4 g (0.024 mol) epibromohydrin, the mixture was heated for a further 4 hours at 160 °C. The excess epibromohydrin was distilled off under reduced pressure. Yield of 1,3,5-tris[(1,3-dibromo-2-propoxy)methyl]benzene was 37 g (85%).

Example 4

1,3,5-tris[(1,3-dibromo-2-propoxy)methyl]-2,4,6-trimethylbenzene was prepared as follows. A mixture of 9.27 g epibromohydrin, 7.5 g of the 1,3,5-tris-(bromomethyl)-2,4,6-trimethylbenzene prepared according to Example 2 and 10 mg mercury dichloride was heated for 12 hours at 160 °C. The reaction mixture was cooled and washed with pentane. Yield of 1,3,5-tris[(1,3-dibromo-2-propoxy)methyl]-2,4,6-trimethylbenzene was 13,2 g (100%).

Example 5

1,3,5-tris[(1,3-bis(bis(2-methoxyphenyl)phosphino)-2-propoxy)methyl]benzene was prepared as follows. (All steps were carried out under argon.) To 125 ml dry ammonia which was maintained at -78 °C was added 0.4 g (17.5 mmol) sodium, 3.1 g (8.75 mmol) tris(2-methoxyphenyl)phosphine and 12.5 ml tetrahydrofuran dried over sodium. After stirring for 6 hours at -78 °C, 0.467 g (8.75 mmol) ammonium chloride was added. After 30 minutes 4.3 mmol of the 1,3,5-tris[(1,3-dibromo-2-propoxy)methyl]benzene prepared according to Example 3 and dissolved in 50 ml tetrahydrofuran was added. After evaporation of ammonia, the reaction mixture was purified as follows (purification procedure 1). The solvent was removed under reduced pressure. The solid material remaining behind was dissolved in 50 ml dichloromethane and washed with 50 ml of a 5% aqueous solution of ammonium chloride. The solvent was then removed and 50 ml tetrahydrofuran was added. The solution was filtered, concentrated to 10 ml and 50 ml methanol was added, causing the hexakisphosphine to precipitate. Yield of 1,3,5-tris[(1,3-bis(2-methoxyphenyl) phosphino)-2-propoxy)methyl]benzene was 1.2 g (48%).

If desired, the reaction mixture can also be purified as follows (purification procedure 2). 50 ml diethyl ether is added to the reaction mixture and the suspension is centrifuged for 5 minutes at 3300 rpm. After concentration of the solution, methanol is added, causing the hexakisphosphine to precipitate.

Example 6

1,3,5-tris[(1,3-bis(bis(2-methoxyphenyl)phosphino)-2-propoxy)methyl]-2,4,6-trimethylbenzene was prepared in substantially the same way as the 1,3,5-tris[(1,3-bis(bis(2-methoxyphenyl)phosphino)-2-propoxy)methyl]benzene in Example 5, but using the 1,3,5-tris[(1,3-dibromo-2-propoxy)methyl]-2,4,6-trimethylbenzene prepared according to Example 4 instead of 1,3,5-tris[(1,3-dibromo-2-propoxy)methyl]benzene. Yield of 1,3,5-tris[(1,3-bis(bis(2-methoxyphenyl)phosphino)-2-propoxy)methyl]-2,4,6-trimethylbenzene was 95%.

Example 7

A carbon monoxide/ethene/propene terpolymer was prepared as follows. Into a stirred autoclave with a capacity of 300 ml was introduced a catalyst solution comprising:
135 ml methanol,
4 ml acetone,
0.009 mmol palladium acetate,
0.19 mmol trifluoroacetic acid, and
0.01 mmol 1,3-bis[bis(2-methoxyphenyl)phosphino]propane.

The air in the autoclave was driven out by pressurizing the autoclave with carbon monoxide to a pressure of 50 bar followed by releasing the pressure and then repeating these steps two more times. After the autoclave had been brought to 82 °C, 25 bar carbon monoxide was forced in, then 10 bar propene and finally 15 bar ethene. During the polymerization the pressure in the autoclave was maintained at 52 bar by forcing in a 1:1 carbon monoxide/ethene mixture. After 8.2 hours the polymerization was terminated by cooling to room temperature and releasing the pressure.

A polymer suspension was obtained which contained 12.3 g terpolymer. 12.4 g terpolymer remained behind in the autoclave, so that the reactor fouling in this case was

$$\frac{12.4}{12.3 + 12.4} \times 100 = 50\%$$

The reaction rate was 3.1 kg terpolymer/(g palladium.hour).

Example 8

A carbon monoxide/ethene/propene terpolymer was prepared in substantially the same way as in Example 7, but with the following differences:
a) the catalyst solution contained 0.01 mmol [(1,3-bis(bis(2-methoxyphenyl)phosphino)-2-propoxy)methyl]benzene instead of 1,3-bis[bis(2-methoxyphenyl)phosphino]propane,
b) the reaction temperature was 77 °C instead of 82 °C, and
c) the reaction time was 3.5 hours instead of 8.2 hours.

A polymer suspension was obtained which contained 10.0 g terpolymer. 7.9 g terpolymer remained behind in the autoclave, so that the reactor fouling in this case was 44%. The reaction rate was 5.3 kg terpolymer/(g palladium.hour).

## Example 9

A carbon monoxide/ethene/propene terpolymer was prepared in substantially the same way as in Example 7, but with the following differences:

a) the catalyst solution contained 0.005 mmol 1,4-bis[(1,3-bis(bis(2-methoxyphenyl)phosphino)-2-propoxy)methyl]benzene instead of 1,3-bis[bis(2-methoxyphenyl)phosphino]propane,

b) the reaction temperature was 80 °C instead of 82 °C, and

c) the reaction time was 2.7 hours instead of 8.2 hours.

A polymer suspension was obtained which contained 12.8 g terpolymer. 6.1 g terpolymer remained behind in the autoclave, so that the reactor fouling in this case was 32%. The reaction rate was 7.3 kg terpolymer/(g palladium.hour).

## Example 10

A carbon monoxide/ethene/propene terpolymer was prepared in substantially the same way as in Example 7, but with the following differences:

a) the catalyst solution contained 0.003 mmol of the 1,3,5-tris[(1,3-bis(bis(2-methoxyphenyl)phosphino)-2-propoxy)methyl]benzene prepared according to Example 5 instead of 1,3-bis[bis(2-methoxyphenyl)phosphino]propane,

b) the reaction temperature was 80 °C instead of 82 °C, and

c) the reaction time was 2.7 hours instead of 8.2 hours.

A polymer suspension was obtained which contained 8.7 g terpolymer. 0.3 g terpolymer remained behind in the autoclave, so that the reactor fouling in this case was 3%. The reaction rate was 3.5 kg terpolymer/(g palladium.hour).

## Example 11

A carbon monoxide/ethene/propene terpolymer was prepared in substantially the same way as in Example 7, but with the following differences:

a) the catalyst solution contained 0.003 mmol of the 1,3,5-tris[(1,3-bis(bis(2-methoxyphenyl)phosphino)-2-propoxy)methyl]-2,4,6- trimethylbenzene prepared according to Example 6 instead of 1,3-bis[bis(2-methoxyphenyl)phosphino]propane,

b) the reaction temperature was 85 °C instead of 82 °C, and

c) the reaction time was 5.1 hours instead of 8.2 hours.

A polymer suspension was obtained which contained 13.7 g terpolymer. 1.5 g terpolymer remained behind in the autoclave, so that the reactor fouling in this case was 10%. The reaction rate was 3.1 kg terpolymer/(g palladium.hour).

Of the Examples 1-11, Examples 3-6, 10 and 11 are according to the invention. Examples 3-4 relate to the preparation of novel hexahalogen compounds which were used in the preparation of the novel hexakisphosphines according to Examples 5 and 6. Examples 10 and 11 relate to the preparation of carbon monoxide/ethene/propene terpolymers using the novel catalyst compositions of the invention. Examples 1, 2 and 7-9 fall outside the scope of the invention. Examples 1 and 2 relate to the preparation of trihalides which were used in the preparation of the novel hexahalides according to Examples 3 and 4. Examples 7-9 relate to the preparation of carbon monoxide-/ethene/propene terpolymers using catalyst compositions comprising a bis- or tetrakisphosphine.

It was established by $^{13}$C-NMR analysis that the carbon monoxide/ethene/propene terpolymers prepared according to Examples 7-11 consisted of linear chains in which the units from carbon monoxide on the one hand and the units from ethene and propene on the other hand occurred in an alternating arrangement. The units from ethene and propene occurred in the polymer chains in a random arrangement.

The favourable influence on the behaviour of the catalyst compositions which occurs if a bisphosphine of the general formula $(R^1R^2P)_2R^3$ or a tetrakisphosphine of the general formula $(R^1R^2P)_4R^4$ is replaced by a hexakisphosphine of the general formula $(R^1R^2P)_6R^5$ can be seen by comparison of the results of Examples 7-9 with those of Examples 10 and 11 (a reduction of reactor fouling from 32-50% to 3-10%).

## Claims

### Claims for the following Contracting States : BE, CH, LI, DE, FR, GB, IT, NL,

1. Catalyst compositions, characterized in that they contain a Group VIII metal and a hexakisphosphine of the general formula $(R^1R^2P)_6R^5$ in which $R^1$ and $R^2$ represent identical or different optionally polar-substituted hydrocarbyl groups and in which $R^5$ is a hexavalent organic linking group which connects the six phosphorus atoms with each other and has a structure such that there are at least two carbon atoms between each two phosphorus atoms.

2. Catalyst compositions according to claim 1, characterized in that they contain palladium as Group VIII metal.

3. Catalyst compositions according to claim 1 or 2, characterized in that they additionally contain an anion of an acid with a pKa of less than 4, in a quantity of 1-100 mol per g.atom Group VIII metal.

4. Catalyst compositions according to one or more of claims 1-3, characterized in that they comprise the hexakisphosphine in a quantity of 0.1-1.0 mol per g.atom Group VIII metal.

5. Catalyst compositions according to one or more of claims 1-4, characterized in that they comprise a hexakisphosphine in which the $R^1$ and $R^2$ groups are identical aryl groups which comprise at least one alkoxy substituent in the ortho position relative to the phosphorus atom to which the aryl group is linked.

6. Catalyst compositions according to claim 5, characterized in that they comprise a hexakisphosphine in which the $R^1$ and $R^2$ groups are 2-methoxyphenyl groups.

7. Catalyst compositions according to one or more of claims 1-6, characterized in that they comprise a hexakisphosphine in which the $R^5$ linking group consists of a benzene ring containing as substituent at the 1-, 3- and 5-positions a (-CH$_2$)$_2$CH-O-

   $\overset{*}{C}$ H$_2$- group which is linked to the benzene ring via the carbon atom labelled with * and which benzene ring optionally contains methyl groups as substituents at the 2-, 4- and 6-positions.

8. Process for the preparation of polymers, characterized in that a mixture of carbon monoxide with one or more olefinically unsaturated compounds is contacted at elevated temperature and pressure with a catalyst composition according to one or more of claims 1-7, which is dissolved in a diluent in which the polymers are insoluble or almost insoluble, such as methanol.

9. Process according to claim 8, characterized in that it is carried out at a temperature of 25 to 150 °C, a pressure of 2 to 150 bar and molar ratio of the olefinically unsaturated compounds relative to carbon monoxide in the mixture to be polymerized of 10:1 to 1:10 and that per mol of olefinically unsaturated compound to be polymerized a quantity of catalyst composition is used which contains $10^{-7}$ to $10^{-3}$ g.atom Group VIII metal.

10. Hexakisphosphines of the general formula [(R$^6$)$_2$P]$_6$R$^5$ in which R$^6$ represents an aryl group containing at least one alkoxy substituent at the ortho position relative to the phosphorus atom to which the aryl group is linked and in which R$^5$ is a hexavalent organic linking group linking the six phosphorus atoms to each other and which consists of a benzene ring containing as substituents at the 1-, 3- and 5-positions a (-CH$_2$)$_2$CH-O-

   $\overset{*}{C}$ H$_2$- group which is linked to the benzene ring via the carbon atom labelled with * and which benzene ring optionally contains methyl groups as substituents at the 2-, 4- and 6-positions.

11. Hexakisphosphines according to claim 10, characterized by being selected from the group consisting of 1,3,5-tris[(1,3-bis(bis(2methoxyphenyl)phosphino)-2-propoxy)methyl]benzene and 1,3,5-tris[(1,3-bis(bis(2-methoxyphenyl)phosphino)-2-propoxy)methyl]-2,4,6-trimethylbenzene.

12. Hexahalo compound suitable for the preparation of the hexakisphosphines according to claim 10, characterized by being selected from the group consisting of 1,3,5-tris[(1,3-dibromo-2-propoxy)methyl]benzene and 1,3,5-tris[(1,3-dibromo-2-propoxy)methyl]-2,4,6-trimethylbenzene.

13. Process for the preparation of the hexahalo compounds according to claim 12, characterized in that epibromohydrin is reacted with 1,3,5-tris(bromomethyl)benzene or with 1,3,5-tris(bromomethyl)-2,4,6-trimethylbenzene, respectively.

14. Process for the preparation of hexakisphosphines according to claim 10, characterized in that an alkali metal diaryl phosphide with the general formula MP(R$^6$)$_2$ in which M represents an alkali metal is reacted with a hexahalogen compound consisting of a benzene ring containing as substituent at the 1-, 3- and 5-positions a (CH$_2$X)$_2$CH-O-CH$_2$- group in which X represents a halogen and which benzene ring optionally contains a methyl group as substituent at the 2-, 4- and 6-positions.

15. Process according to claim 14, characterized in that the alkali metal diaryl phosphide required for the preparation of the hexakisphosphines has been obtained by the reaction in liquid ammonia of an alkali metal M with a trisphosphine of the general formula (R$^6$)$_3$P.

16. Process according to claim 14, characterized in that the hexahalo compound needed for the preparation of the hexakisphosphine has been obtained according to claim 13.

**Claims for the following Contracting State : ES**

1. Process for the preparation of polymers, characterized in that a mixture of carbon monoxide with one or more olefinically unsaturated compounds is contacted at elevated temperature and pressure with a catalyst composition which is dissolved in a diluent in which the polymers are insoluble or almost insoluble, and which catalyst composition contains a Group VIII metal and a hexakisphosphine of the general formula $(R^1R^2P)_6R^5$ in which $R^1$ and $R^2$ represent identical or different optionally polar-substituted hydrocarbyl groups and in which $R^5$ is a hexavalent organic linking group which connects the six phosphorus atoms with each other and has a structure such that there are at least two carbon atoms between each two phosphorus atoms.

2. Process according to claim 1, characterized in that the catalyst composition contains palladium as Group VIII metal.

3. Process according to claim 1 or 2, characterized in that the catalyst composition additionally contains an anion of an acid with a pKa of less than 4, in a quantity of 1-100 mol per g.atom Group VIII metal.

4. Process according to one or more of claims 1-3, characterized in that the catalyst composition comprises the hexakisphosphine in a quantity of 0.1-1.0 mol per g.atom Group VIII metal.

5. Process according to one or more of claims 1-4, characterized in that the catalyst composition comprises a hexakisphosphine in which the $R^1$ and $R^2$ groups are identical aryl groups which comprise at least one alkoxy substituent in the ortho position relative to the phosphorus atom to which the aryl group is linked.

6. Process according to claim 5, characterized in that the catalyst composition comprises a hexakisphosphine in which the $R^1$ and $R^2$ groups are 2-methoxyphenyl groups.

7. Process according to one or more of claims 1-6, characterized in that the catalyst composition comprises a hexakisphosphine in which the $R^5$ linking group consists of a benzene ring containing as substituent at the 1-, 3- and 5-positions a

$(-CH_2)_2CH-O-\overset{*}{C}H_2-$ group which is linked to the benzene ring via the carbon atom labelled with * and which benzene ring optionally contains methyl groups as substituents at the 2-, 4- and 6-positions.

8. Process according to one or more of claims 1-7, characterized in that the diluent is methanol.

9. Process according to one or more of claims 1-8, characterized in that it is carried out at a temperature of 25 to 150 °C, a pressure of 2 to 150 bar and molar ratio of the olefinically unsaturated compounds relative to carbon monoxide in the mixture to be polymerized of 10:1 to 1:10 and that per mol of olefinically unsaturated compound to be polymerized a quantity of catalyst composition is used which contains $10^{-7}$ to $10^{-3}$ g.atom Group VIII metal.

10. Process for the preparation of hexahalo compounds selected from the group consisting of 1,3,5-tris[(1,3-dibromo-2-propoxy)methyl)benzene and 1,3,5-tris[(1,3-dibromo-2-propoxy)methyl]-2,4,6-trimethylbenzene, characterized in that epibromohydrin is reacted with 1,3,5-tris(bromomethyl)benzene or with 1,3,5-tris(bromomethyl)-2,4,6-trimethylbenzene, respectively.

11. Process for the preparation of hexakisphosphines of the general formula $[(R^6)_2P]_6R^5$ in which $R^6$ represents an aryl group containing at least one alkoxy substituent at the ortho position relative to the phosphorus-atom to which the aryl group is linked and in which $R^5$ is a hexavalent organic linking group linking the six phosphorus atoms to each other and which consists of a benzene ring containing as substituents at the 1-, 3- and 5-positions a $(-CH_2)_2CH-O-\overset{*}{C}H_2-$ group which is linked to the benzene ring via the carbon atom labelled with * and which benzene ring optionally contains methyl groups as substituents at the 2-, 4- and 6-positions, characterized in that an alkali metal diaryl phosphide with the general formula $MP(R^6)_2$ in which M represents an alkali metal is reacted with a hexahalogen compound consisting of a benzene ring containing as substituent at the 1-, 3- and 5-positions a $(CH_2X)_2CH-O-CH_2-$ group in which X represents a halogen and which benzene ring optionally contains a methyl group as substituent at the 2-, 4- and 6-positions.

12. Process according to claim 11, characterized in that the alkali metal diaryl phosphide required for the preparation of the hexakisphosphines has been obtained by the reaction in liquid ammonia of an alkali metal M with a trisphosphine of the general formula $(R^6)_3P$.

13. Process according to claim 11 or 12, characterized in that a hexakisphosphine is prepared which is selected from the group consisting of 1,3,5-tris[(1,3-bis(bis(2-methoxphenyl)phosphino)-2-propoxy)methyl]benzene and 1,3,5-tris[(1,3-bis(bis(2-methoxyphenyl)phosphino)-2-propoxy)methyl]-2,4,6-trimethylbenzene.

14. Process according to claim 11, characterized in that the hexahalo compound needed for the preparation of the hexakisphosphine has been obtained according to claim 10.

**Patentansprüche**

**Patentansprüche für folgende Vertragsstaaten : BE, CH, LI, DE, FR, GB, IT, NL,**

1. Katalysatorzusammensetzungen, dadurch gekennzeichnet, daß sie ein Metall der Gruppe VIII und ein Hexakisphosphin der allgemeinen Formel $(R^1R^2P)_6R^5$ enthalten, in welcher $R^1$ und $R^2$ identische oder verschiedene, gegebenenfalls polar-substituierte Kohlenwasserstoffgruppen darstellen, und in welcher $R^5$ eine sechswertige organische Verbindungsgruppe ist, welche die 6 Phosphoratome miteinander verbindet und eine Struktur derart aufweist ,daß mindestens zwei Kohlenstoffatome zwischen jeweils zwei Phosphoratomen vorliegen.

2. Katalysatorzusammensetzungen nach Anspruch 1, dadurch gekennzeichnet, daß sie Palladium als Metall der Gruppe VIII enthalten.

3. Katalysatorzusammensetzungen nach Anspruch 1 oder 2, dadurch gekennzeichnet, daß sie zusätzlich ein Anion einer Säure mit einem pKa-Wert von weniger als 4 in einer Menge von 1 bis 100 Mol je Grammatom des Metalls der Gruppe VIII enthalten.

4. Katalysatorzusammensetzungen nach einem oder mehreren der Ansprüche 1 bis 3, dadurch gekennzeichnet, daß sie das Hexakisphosphin in einer Menge von 0,1 bis 1,0 Mol je Grammatom des Metalls der Gruppe VIII enthalten.

5. Katalysatorzusammensetzungen nach einem oder mehreren der Ansprüche 1 bis 4, dadurch gekennzeichnet, daß sie ein Hexakisphosphin enthalten, in welchem die $R^1$- und $R^2$-Gruppen identische Arylgruppen sind, welche mindestens einen Alkoxysubstituenten in der ortho-Stellung in bezug auf das Phosphoratom, an welches die Arylgruppe gebunden ist, enthalten.

6. Katalysatorzusammensetzungen nach Anspruch 5, dadurch gekennzeichnet , daß sie ein Hexakisphosphin enthalten, in welchem die $R^1$- und $R^2$-Gruppen 2-Methoxyphenylgruppen sind.

7. Katalysatorzusammensetzungen nach einem oder mehreren der Ansprüche 1 bis 6, dadurch gekennzeichnet, daß sie ein Hexakisphosphin enthalten, in welchem die $R^5$-Verbindungsgruppe aus einem Benzolring besteht, der als Substituent in den 1-, 3- und 5-Positionen eine $(-CH_2)_2$ CH-O-$\overset{*}{C}$H$_2$-Gruppe enthält, welche über das Kohlenstoffatom, das mit * gekennzeichnet ist, an den Benzolring gebunden ist, und welcher Benzolring gegebenenfalls Methylgruppen als Substituenten in den 2-,4- und 6-Positionen enthält.

8. Verfahren zur Herstellung von Polymeren, dadurch gekennzeichnet, daß eine Mischung von Kohlenmonoxid mit einer oder mehreren olefinisch ungesättigten Verbindung(en) bei erhöhter Temperatur und erhöhtem Druck mit einer Katalysatorzusammensetzung nach einem oder mehreren der Ansprüche 1 bis 7 kontaktiert wird, welche in einem Verdünnungsmittel gelöst ist, in welchem die Polymere nicht löslich sind oder kaum löslich sind, wie z.B. Methanol.

9. Verfahren nach Anspruch 8, dadurch gekennzeichnet, daß es bei einer Temperatur von 25 bis 150°C, einem Druck von 2 bis 150 bar und einem Molverhältnis der olefinisch ungesättigten Verbindungen in bezug auf das Kohlenmonoxid in der zu polymerisierenden Mischung von 10:1 bis 1:10 durchgeführt wird, und daß je Mol zu polymerisierender olefinisch ungesättigter Verbindung eine Menge der Katalysatorzusammensetzung eingesetzt wird, welche $10^{-7}$ bis $10^{-3}$ Grammatom des Metalls der Gruppe VIII enthält.

10. Hexakisphosphine der allgemeinen Formel $[(R^6)_2P]_6R^5$, in welcher $R^6$ eine Arylgruppe darstellt, die mindestens einen Alkoxysubstituenten in der ortho-Position in bezug auf das Phosphoratom, an welches die Arylgruppe gebunden ist, enthält, und in welcher $R^5$ eine sechswertige organische Verbindungsgruppe ist, welche die 6 Phosphoratome aneinander bindet und welche aus einem Benzolring besteht, der als Substituenten in der 1-, 3- und 5 -Position eine $(-CH_2)_2$ CH-O-$\overset{*}{C}$H$_2$-Gruppe enthält, die über das Kohlenstoffatom, das mit * gekennzeichnet ist, an den Benzolring gebunden ist, und welcher Benzolring gegebenenfalls Methylgruppen als Substituenten in den 2-,4- und 6- Positionen enthält.

11. Hexakisphosphine nach Anspruch 10, dadurch gekennzeichnet, daß sie ausgewählt sind aus der Gruppe, bestehend aus 1,3,5-Tris[(1,3-bis(bis(2-methoxyphenyl)-phosphino)-2-propoxy)methyl]benzol und 1,3,5-Tris-[(1,3-bis(bis(2-methoxyphenyl)phosphino)-2-propoxy)-methyl]-2,4,6-trimethylbenzol.

12. Hexahalogenverbindung, welche für die Herstellung der Hexakisphosphine nach Anspruch 10 geeignet ist, dadurch gekennzeichnet, daß sie ausgewählt ist aus der Gruppe, bestehend aus 1,3,5-Tris[(1,3-dibrom-2-propoxy)-methyl]benzol und 1,3,5-Tris [(1,3-dibrom-2-propoxy)-methyl]-2,4,6-trimethylbenzol.

13. Verfahren zur Herstellung der Hexahalogenverbindungen nach Anspruch 12, dadurch gekennzeichnet, daß Epibromhydrin mit 1,3,5-Tris (brommethyl)-benzol bzw. mit 1,3,5-Tris(brommethyl)-2,4,6-trimethylbenzol umgesetzt wird.

14. Verfahren zur Herstellung von Hexakisphosphinen nach Anspruch 10, dadurch gekennzeichnet, daß ein Alkalimetalldiarylphosphid der allgemeinen Formel $MP(R^6)_2$ , in welcher M ein Alkalimetall darstellt, mit einer Hexahalogenverbindung umgesetzt wird, welche aus einem Benzolring besteht, der als Substituenten in den 1-, 3- und 5-Positionen eine $(CH_2X)_2CH-O-CH_2$-Gruppe enthält, in welcher X ein Halogen bedeutet, und welcher Benzolring gegebenenfalls eine Methylgruppe als Substituent in den 2-, 4- und 6-Positionen enthält.

15. Verfahren nach Anspruch 14, dadurch gekennzeichnet, daß das Alkalimetalldiarylphosphid, das zur Herstellung der Hexakisphosphine benötigt wird, durch Umsetzung in flüssigem Ammoniak eines Alkalimetalls M mit einem Triphosphin der allgemeinen Formel $(R^6)_3P$ erhalten wird.

16. Verfahren nach Anspruch 14, dadurch gekennzeichnet, daß die Hexahalogenverbindung, die für die Herstellung der Hexakisphosphine nötig ist, wie in Anspruch 13 beschrieben erhalten worden ist.

**Patentansprüche für folgenden Vertragsstaat : ES**

1. Verfahren zur Herstellung von Polymeren, dadurch gekennzeichnet, daß eine Mischung von Kohlenmonoxid mit einer oder mehreren olefinisch ungesättigten Verbindung(en) bei erhöhter Temperatur und erhöhtem Druck mit einer Katalysatorzusammensetzung kontaktiert wird, welche in einem Verdünnungsmittel gelöst ist, in welchem die Polymere nicht löslich oder kaum löslich sind, und welche Katalysatorzusammensetzung ein Metall der Gruppe VIII und ein Hexakisphosphin der allgemeinen Formel $(R^1R^2P)_6R^5$ enthält, wobei $R^1$ und $R^2$ identische oder verschiedene, gegebenenfalls polar-substituierte Kohlenwasserstoffgruppen darstellen und wobei $R^5$ eine sechswertige organische Ver-

bindungsgruppe ist, welche die 6 Phosphoratome miteinander verbindet und eine Struktur derart aufweist, daß mindestens 2 Kohlenstoffatome zwischen jeweils zwei Phosphoratomen vorliegen.

2. Verfahren nach Anspruch 1, dadurch gekennzeichnet, daß die Katalysatorzusammensetzung Palladium als Metall der Gruppe VIII enthält.

3. Verfahren nach Anspruch 1 oder 2, dadurch gekennzeichnet, daß die Katalysatorzusammensetzung außerdem ein Anion einer Säure mit einem pKa-Wert von weniger als 4 in einer Menge von 1 bis 100 Mol je Grammatom des Metalls der Gruppe VIII enthält.

4. Verfahren nach Anspruch 1 bis 3 dadurch gekennzeichnet, daß die Katalysatorzusammensetzung das Hexakisphosphin in einer Menge von 0,1 bis 1,0 Mol je Grammatom des Metalls der Gruppe VIII enthält.

5. Verfahren nach einem oder mehreren der Ansprüche 1 bis 4, dadurch gekennzeichnet, daß die Katalysatorzusammensetzung ein Hexakisphosphin umfaßt, in welchem die Gruppen $R^1$ und $R^2$ identische Arylgruppen sind, welche mindestens einen Alkoxysubstituenten in der ortho-Position in bezug auf das Phosphoratom, an welches die Arylgruppe gebunden ist, enthalten.

6. Verfahren nach Anspruch 5, dadurch gekennzeichnet, daß die Katalysatorzusammensetzung ein Hexakisphosphin enthält, in welchem die $R^1$- und $R^2$-Gruppen 2-Methoxyphenylgruppen sind.

7. Verfahren nach einem oder mehreren der Ansprüche 1 bis 6, dadurch gekennzeichnet, daß die Katalysatorzusammensetzungen ein Hexakisphosphin enthält, in welchem die $R^5$-Verbindungsgruppe aus einem Benzolring besteht, der als Substituenten in den 1-, 3- und 5- Positionen eine $(-CH_2)_2CH-O-\overset{*}{C}H_2$-Gruppe enthält, die über das mit * gekennzeichnete Kohlenstoffatom an den Benzolring gebunden ist, und welcher Benzolring gegebenenfalls Methylgruppen als Substituenten in den 2-,4- und 6-Positionen enthält.

8. Verfahren nach einem oder mehreren der Ansprüche 1 bis 7, dadurch gekennzeichnet, daß das Verdünnungsmittel Methanol ist.

9. Verfahren nach einem oder mehreren der Ansprüche 1 bis 8 , dadurch gekennzeichnet, daß es bei einer Temperatur von 25 bis 150°C, einem Druck von 2 bis 150 bar und einem Molverhältnis der olefinisch ungesättigten Verbindungen in bezug auf das Kohlenmonoxid in der zu polymerisierenden Mischung von 10:1 bis 1:10 durchgeführt wird, und daß je Mol olefinisch ungesättigter zu polymerisierender Verbindung eine Menge einer Katalysatorzusammensetzung eingesetzt wird, welche $10^{-7}$ bis $10^{-3}$ Grammatom des Metalls der Gruppe VIII enthält.

10. Verfahren zur Herstellung von Hexahalogenverbindungen, ausgewählt aus der Gruppe, bestehend aus 1,3,5-Tris[(1,3-dibrom-2-propoxy)methyl]benzol und 1,3,5-Tris((1,3-dibrom-2-propoxy)methyl]-2,4,6-trimethylbenzol, dadurch gekennzeichnet, daß Epibromhydrin mit 1,3,5-Tris(brommethyl)benzol bzw.mit 1,3,5-Tris-(brommethyl)-2,4,6-trimethylbenzol umgesetzt wird.

11. Verfahren zur Herstellung von Hexakisphosphinen der allgemeinen Formel $[(R^6)_2P]_6R^5$, in welcher $R^6$ eine Arylgruppe ist, welche mindestens einen Alkoxysubstituenten in der ortho-Position in bezug auf das Phosphoratom, an welches die Arylgruppe gebunden ist, enthält, und in welcher $R^5$ eine sechswertige organische Verbindungsgruppe ist, die die 6 Phosphoratome untereinander verbindet und aus einem Benzolring besteht, der als Substituenten in den 1-, 3- und 5-Positionen eine $(-CH_2)_2-CH-O-\overset{*}{C}H_2$-Gruppe enthält, die über das mit * gekennzeichnete Kolenstoffatom an den Benzolring gebunden ist, und welcher Benzolring gegebenenfalls Methylgruppen als Substituenten in den 2-, 4- und 6-Positionen enthält, dadurch gekennzeichnet, daß ein Alkalimetalldiarylphosphid mit der allgemeinen Formel $MP(R^6)_2$, in welcher M ein Alkalimetall darstellt, mit einer Hexahalogenverbindung umgesetzt wird, die aus einem Benzolring besteht, der als Substituenten in den 1-, 3- und 5- Positionen eine $(CH_2X)_2CH-O-CH_2$-Gruppe enthält, in welcher X ein Halogen ist, und welcher Benzolring gegebenenfalls eine Methylgruppe als Substituenten in den 2-,4- und 6- Positionen umfaßt.

12. Verfahren nach Anspruch 11, dadurch gekennzeichnet, daß das für die Herstellung der Hexakisphosphine notwendige Alkalimetalldiarylphosphid durch Umsetzung in flüssigem Ammoniak eines Alkalimetalls M mit einem Triphosphin der allgemeinen Formel $(R_6)_3P$ erhalten worden ist.

13. Verfahren nach Anspruch 11 oder 12, dadurch gekennzeichnet, daß ein Hexakisphosphin hergestellt wird, das ausgewählt ist aus der Gruppe, bestehend aus 1,3,5-Tris[(1,3-bis(bis(2-methoxyphenyl)phosphino-2-propoxy)methyl]benzol und 1,3,5-Tris[(1,3-bis(bis(2-methoxyphenyl)phosphino)-2-propoxy)methyl]-2,4,6-trimethylbenzol.

14. Verfahren nach Anspruch 11, dadurch gekennzeichnet, daß die Hexahalogenverbindung, die für die Herstellung der Hexakisphosphine notwendig ist, gemäß Anspruch 10 erhalten worden ist.

## Revendications

### Revendications pour les Etats contractants suivants : BE, CH, LI, DE, FR, GB, IT, NL,

1. Compositions catalytiques, caractérisées en ce qu'elles contiennent un métal du groupe VIII et une hexakisphosphine de la formule générale $(R^1R^2P)_6R^5$ dans laquelle $R^1$ et $R^2$ représentent des groupes hydrocarbyle identiques ou différents éventuellement substitués par des substituants polaires et $R^5$ est un groupe de jonction organique hexavalent qui relie entre eux les six atomes de phosphore et a une structure telle qu'il y ait au moins deux atomes de carbone entre chaque paire d'atomes de phosphore.

2. Compositions catalytiques selon la revendication 1, caractérisées en ce qu'elles contiennent du palladium comme métal du groupe VIII.

3. Compositions catalytiques selon la revendication 1 ou 2, caractérisées en ce qu'elles contiennent en outre un anion d'un acide d'un pKa de moins de 4, dans une quantité de 1-100 moles par atome-gramme de métal du groupe VIII.

4. Compositions catalytiques selon une ou plusieurs des revendications 1-3, caractérisées en ce qu'elles comprennent l'hexakisphosphine à raison de 0,1-1,0 mole par atome-gramme de métal du groupe VIII.

5. Compositions catalytiques selon une ou plusieurs des revendications 1-4, caractérisées en ce qu'elles comprennent une hexakisphosphine dans laquelle les groupes $R^1$ et $R^2$ sont des groupes aryle identiques qui comprennent au moins un substituant alcoxy dans la position ortho par rapport à l'atome de phosphore auquel le groupe aryle est lié.

6. Compositions catalytiques selon la revendication 5, caractérisées en ce qu'elles comprennent une hexakisphosphine dans laquelle les groupes $R^1$ et $R^2$ sont des groupes 2-méthoxyphényle.

7. Compositions catalytiques selon une ou plusieurs des revendications 1-6, caractérisées en ce qu'elles comprennent une hexakisphosphine dans laquelle le groupe de jonction $R^5$ consite en un noyau de benzène contenant comme substituant aux positions 1, 3 et 5 un groupe (-CH$_2$)$_2$CH-O- $\overset{*}{C}$ H$_2$- qui est lié au noyau de benzène par l'atome de carbone marqué par *, le noyau de benzène contenant éventuellement des groupes méthyle comme substituants aux positions 2, 4 et 6.

8. Procédé de préparation de polymères, caractérisé en ce qu'un mélange d'oxyde de carbone avec un ou plusieurs composés oléfiniquement insaturés est mis en contact à température et pression élevées avec une composition catalytique selon une ou plusieurs des revendications 1-7, qui est dissoute dans un diluant dans lequel les polymères sont insolubles ou presque insolubles, comme le méthanol.

9. Procédé selon la revendication 8, caractérisé en ce qu'il est mis en oeuvre à une température de 25 à 150°C, une pression de 2 à 150 bars et un rapport molaire des composés oléfiniquement insaturés à l'oxyde de carbone dans le mélange à polymériser compris entre 1:10 et 10:1 et que, par mole de composé oléfiniquement insaturé à polymériser, on utilise une quantité de composition catalytique qui contient $10^{-7}$ à $10^{-3}$ atome-gramme de métal du groupe VIII.

10. Hexakisphosphines de la formule générale ((R$^6$)$_2$P)$_6$R$^5$ dans laquelle R$^6$ représente un groupe aryle contenant au moins un substituant alcoxy à la position ortho par rapport à l'atome de phosphore auquel le groupe aryle est lié et R$^5$ est un groupe de jonction organique hexavalent reliant entre eux les six atomes de phosphore et qui consiste en un noyau de benzène comme substituants aux positions 1, 3 et 5 un groupe (-CH$_2$)$_2$CH-O- $\overset{*}{C}$ H$_2$- qui est lié au noyau de benzène par l'atome de carbone marqué par *, le noyeu de benzène contenant éventuellement des groupes méthyle comme substituants aux positions 2, 4 et 6.

11. Hexakisphosphines selon la revendication 10, caractérisées en ce qu'elles sont choisies dans le groupe constitué par le 1,3,5-tris((1,3-bis(bis(2-méthoxyphényl)phosphino)-2-propoxy)méthyl)benzène et le 1,3,5-tris((1,3-bis(bis(2-méthoxyphényl)phosphino-2-propoxy)méthyl)-2,4,6-triméthylbenzène.

12. Composé hexahalogéné utilisable pour la préparation des hexakisphosphines selon la revendication 10, caractérisé en ce qu'il est choisi dans le groupe constitué par le 1,3,5-tris((1,3-dibromo-2-propoxy)méthyl)benzène et le 1,3,5-tris((1,3-dibromo-2-propoxy)méthyl)-2,4,6-triméthylbenzène.

13. Procédé pour la préparation des composés hexahalogénés selon la revendication 12, caractérisé en ce qu'on fait réagir l'épibromhydrine avec le 1,3,5-tris(bromoéthyl)benzène ou avec le 1,3,5-tris(bromométhyl)-2,4,6-triméthylbenzène, respectivement.

14. Procédé pour la préparation d'hexakisphosphines selon la revendication 10, caractérisé en ce qu'un diaryl phosphure de métal alcalin de la formule générale MP(R$^6$)$_2$ dans laquelle M représente un métal alcalin est mis à réagir avec un composé hexahalogéné consistant en un noyau de benzène contenant comme substituant aux positions 1, 3 et 5 un groupe (CH$_2$X)$_2$CH-O-CH$_2$-, où X représente un halogène, le noyau de benzène contenant éventuellement un groupe méthyle comme substituant aux positions 2, 4 et 6.

15. Procédé selon la revendication 14, caractérisé en ce que le diaryl phosphure de métal alcalin nécessaire pour la préparation des hexakisphosphines a été obtenu par la réaction dans l'ammoniac liquide d'un métal alcalin M avec une trisphosphine de la formule générale (R$^6$)$_3$P.

16. Procédé selon la revendication 14, caractérisé en ce que le composé hexahalogéné nécessaire pour la préparation de l'hexakisphosphine a été obtenu selon la revendication 13.

**Revendications pour l'Etat contractant suivant : ES**

1. Procédé pour la préparation de polymères, caractérisé en ce qu'un mélange d'oxyde de carbone avec un ou plusieurs composés oléfiniquement insaturés est mis en contact à température et pression élevées avec une composition catalytique qui est dissoute dans un diluant dans lequel les polymères sont insolubles ou presque insolubles, cette composition catalytique contenant un métal du groupe VIII et une hexakisphosphine de la formule générale (R$^1$R$^2$P)$_6$R$^5$ dans laquelle R$^1$ et R$^2$ représentent des groupes hydrocarbyle identiques ou différents éventuellement substitués par des substituants polaires et R$^5$ est un groupe de jonction organique hexavalent qui relie entre eux les six atomes de phosphore et a une structure telle qu'il y ait au moins deux atomes de carbone entre chaque paire d'atomes de phosphore.

**2.** Procédé selon la revendication 1, caractérisé en ce que la composition catalytique contient du palladium comme métal du groupe VIII.

**3.** Procédé selon la revendication 1 ou 2, caractérisé en ce que la composition catalytique contient en outre un anion d'un acide d'un pKa de moins de 4, dans une quantité de 1-100 moles par atome-gramme de métal du groupe VIII.

**4.** Procédé selon une ou plusieurs des revendications 1-3, caractérisé en ce que la composition catalytique comprend l'hexakisphosphine à raison de 0,1-1,0 mole par atome-gramme de métal du groupe VIII.

**5.** Procédé selon une ou plusieurs des revendications 1-4, caractérisé en ce que la composition catalytique comprend une hexakisphosphine dans laquelle les groupes $R^1$ et $R^2$ sont des groupes aryle identiques qui comprennent au moins un substituant alcoxy dans la position ortho par rapport à l'atome de phosphore auquel le le groupe aryle est lié.

**6.** Procédé selon la revendication 5, caractérisé en ce que la composition catalytique comprend une hexakisphosphine dans laquelle les groupes $R^1$ et $R^2$ sont des groupes 2-méthoxyphényle.

**7.** Procédé selon une ou plusieurs des revendications 1-6, caractérisé en ce que la composition catalytique comprend une hexakisphosphine dans laquelle le groupe de jonction $R^5$ consiste en un noyau de benzène contenant comme substituant aux positions 1, 3 et 5 un groupe -$(CH_2)_2$CH-O-$\overset{*}{C}H_2$- qui est lié au noyau de benzène par l'atome de carbone marqué par *, le noyau de benzène contenant éventuellement des groupes méthyle comme substituants aux positions 2, 4 et 6.

**8.** Procédé selon une ou plusieurs des revendications 1-7, caractérisé en ce que le diluant est du méthanol.

**9.** Procédé selon une ou plusieurs des revendications 1-8, caractérisé en ce qu'il est mis en oeuvre à une température de 25 à 150°C, une pression de 2 à 150 bars et un rapport molaire entre les composés oléfiniquement insaturés et l'oxyde de carbone dans le mélange à polymériser de 10:1 à 1:10 et que, par mole de composé oléfiniquement insaturé à polymériser, on utilise une quantité de composition catalytique qui contient de $10^{-7}$ à $10^{-3}$ atome-gramme de métal du groupe VIII.

**10.** Procédé pour la préparation de composés hexahalogénés choisis dans le groupe constitué par le 1,3,5-tris((1,3-dibromo-2-propoxy)méthyl)benzène et le 1,3,5-tris((1,3-dibromo-2-propoxy)méthyl)-2,4,6-triméthylbenzène, caractérisé en ce qu'on fait réagir l'épibromhydrine avec le 1,3,5-tris(bromométhyl)benzène ou avec le 1,3,5-tris(bromométhyl)-2,4,6-triméthylbenzène, respectivement.

**11.** Procédé pour la préparation d'hexakisphosphines de la formule générale $((R^6)_2P)_6R^5$ dans laquelle $R^6$ représente un groupe aryle contenant au moins un substituant alcoxy à la position ortho par rapport à l'atome de phosphore auquel le groupe aryle est lié et $R^5$ est un groupe de jonction organique hexavalent reliant entre eux les six atomes de phosphore et qui consiste en un noyau de benzène contenant comme substituants aux positions 1, 3 et 5 un groupe (-$CH_2)_2$CH-O-$\overset{*}{C}H_2$- qui est lié au noyau de benzène par l'atome de carbone marqué par *, le noyau de benzène contenant éventuellement des groupes méthyle comme substituants aux positions 2, 4 et 6, caractérisé en ce qu'un diaryl phosphure de métal alcalin de la formule générale $MP(R^6)_2$ dans laquelle M représente un métal alcalin est mis à réagir avec un composé hexahalogéné consistant en un noyau de benzène contenant comme substituant aux positions 1, 3 et 5 un groupe $(CH_2X)_2$CH-O-$CH_2$, où X représente un halogène, le noyau de benzène contenant éventuellement un groupe méthyle comme substituant aux positions 2, 4 et 6.

**12.** Procédé selon la revendication 11, caractérisé en ce que le diaryl phosphure de métal alcalin nécessaire pour la préparation des hexakisphosphines a été obtenu par la réaction dans l'ammoniac liquide d'un métal alcalin M avec une trisphosphine de la formule générale $(R^6)_3P$.

**13.** Procédé selon la revendication 11 ou 12, caractérisé en ce qu'on prépare une hexakisphosphine qui est choisie dans le groupe constitué par le 1,3,5-tris-((1,3-bis(bis(2-méthoxyphényl)phosphino)-2-propoxy)méthyl)benzène et le 1,3,5-tris((1,3-bis(bis(2-méthoxyphényl)phosphino)-2-propoxy)méthyl)-2,4,6-triméthylbenzène.

**14.** Procédé selon la revendication 11, caractérisé en ce que le composé hexahalogéné nécessaire pour la préparation de l'hexakisphosphine a été obtenu selon la revendication 10.